# EUROPÄISCHE PATENTSCHRIFT

(11) **EP 1 589 913 B1**
(45) Veröffentlichungstag und Bekanntmachung des Hinweises auf die Patenterteilung: **16.07.2008**
(21) Anmeldenummer: 04703381.6
(22) Anmeldetag: 20.01.2004
(51) Int. Cl.: A61F 5/058, A61F 5/01

(54) **ORTHESENINLAY**
ORTHOPEDIC INLAY
INSERT D'ORTHESE

(30) Priorität: 28.01.2003 DE 10303327
(43) Veröffentlichungstag der Anmeldung: 02.11.2005
(73) Patentinhaber: OPED AG, 6312 Steinhausen (CH)
(72) Erfinder: HASSLER, Andreas, 83101 Rohrdorf (DE); HOPMANN, Gero, 85579 Neubiberg (DE)
(74) Vertreter: Tappe, Hartmut
(86) Internationale Anmeldenummer: PCT/EP2004/000398
(87) Internationale Veröffentlichungsnummer: WO 2004/066892

(56) Entgegenhaltungen:
- WO-A-01/87100
- WO-A-95/31951
- CH-A- 661 204

## Beschreibung

Die vorliegende Erfindung betrifft ein Ortheseninlay zur Anordnung in einer Stützschalenanordnung einer Körperteilorthese mit einem Formkissen zur Anformung an den Körperteil mit zumindest einer in einer gasdichten Hülle ausgebildeten Kissenkammer, die über eine Ventileinrichtung evakuierbar ist und eine Formkörperfüllung aus einer Vielzahl von Formkörpern aufweist, und mit einer Formkissenhülle zur Aufnahme des Formkissens.

Des weiteren betrifft die Erfindung ein Formkissen zur Ausbildung eines Ortheseninlays und eine Formkissenhülle zur Ausbildung eines Orthescninlays.

Ortheseninlays der eingangs genannten Art dienen zur Innenauskleidung von Schalcnanordnungen, wie sie beispielsweise zur Behandlung von Unterschenkelfrakturen sowie insbesondere Achillessehnenrupturen eingesetzt werden. Hierfür wird auf die Veröffentlichung WO 95/31951 verwiesen. Auch bei Unterarmfrakturen oder zur Halswirbel-Stützung bzw. Immobilisierung können derartige Ortheseninlays zum Einsatz kommen. Dem Formkissen kommt dabei zum einen eine auskleidend abpolsternde Funktion zu. Zum anderen soll eine möglichst unmittelbare Einleitung der durch die äußere Stützschalenanordnung aufgebrachten Stützkräfte in das betreffende Körperteil, also beispielsweise den Unterschenkel, ermöglicht werden. Hierzu sind derartige Formkissen mit einer gasdichten Hülle versehen, die zur Ausbildung einer Kissenkammer dient, welche mit einer Formkörperfüllung aus einer Vielzahl von Formkörpern verschen ist. Nach umschließender Anlage des Formkissens an den Unterschenkel erfolgt durch eine Evakuierung der Kissenkammer mittels einer darin vorgesehenen Ventileinrichtung eine Immobilisierung der Formkörper und somit eine Fixierung der durch die Umschließung erreichten Relativanordnung der Formkörper des Formkissens.

Aus dem Vorhergehenden wird deutlich, dass einer sorgfältigen Anpassung des Formkissens, beispielsweise an die Unterschenkel- bzw. Waden- und Fußkontur des Betroffenen, eine erhebliche Bedeutung zukommt, da die hierbei erreichte Relativpositionierung durch den Evakuierungsvorgang fixiert wird und nachfolgend die Effektivität der Stützkrafteinwirkung von der Schalenanordnung auf den Unterschenkel bestimmt. Da die Anpassung des Formkissens an die Unterschenkelkontur ausgehend von einem ebenen Kissenzuschnitt des Formkissens erfolgt, erfordert die Anpassung des ebenen Kissenzuschnitts an die Kontur des betreffenden Körperteils eine entsprechend große Geschicklichkeit. Dasselbe gilt für die Durchführung des eigentlichen Evakuierungsvorgangs, der zur Durchführung von Korrekturen des Kissensitzes teilweise wiederholt durchgeführt werden muss, was mit einer entsprechend wiederholten Betätigung der Ventileinrichtung verbunden ist.

Der vorliegenden Erfindung liegt daher die Aufgabe zugrunde, die vereinfachte Handhabung eines Ortheseninlays der eingangs genannten Art zu ermöglichen.

Diese Aufgabe wird durch ein Ortheseninlay mit den Merkmalen des Anspruchs 1 gelöst.

Bei dem erfindungsgemäßen Ortheseninlay umfasst das Formkissen einen ebenen Kissenzuschnitt mit einem Basisteil mit zumindest zwei Seitenteilen und einem vom Basisteil über eine Einschnürung abgeteilten Laschenteil. Die Formkissenhülle weist zumindest drei jeweils den Seitenteilen des Basisteils und dem Laschenteil zugeordnete Aufnahmebereiche auf, nämlich zwei Seitenteilaufnahmen und eine Laschenteilaufnahme, die benachbart zueinander angeordnet sind, derart, dass die Seitenteile und das Laschenteil des Formkissens nach Aufnahme in den zugeordneten Aufnahmebereichen der Formkissenhülle eine schalenförmige Körperteilaufnahme zur zumindest anteiligen Umhüllung des aufzunehmende Körperteils bilden.

Im Zusammenwirken mit der Formkissenhülle wird demnach bereits im belüfteten Zustand des Formkissens die Ausbildung einer schalenförmigen Körperteilaufnahme ermöglicht, so dass hierdurch schon eine "Grobanpassung" an die Körperteilkontur gegeben ist, die eine nachfolgende, exakte "Feinanpassung" durch eine Evakuierung des Formkissens erheblich erleichtert. Die Einschnürung wirkt dabei zum einen als Scharnierbereich. Zum anderen wird hierdurch verhindert, dass es zu unerwünschten Forinkörperagglomerationen in dem einen und einem Formkörpermangel in dem angrenzenden Bereich kommt.

Der Kissenzuschnitt eines für eine Anordnung am Unterschenkel besonders geeigneten Ortheseninlays kann in einem Fußteil T-förmig ausgebildet sein mit einem der Fußsohle zugeordneten Sohlenteil und einem durch eine Ferseneinschnürung vom Sohlenteil abgeteilten und quer zur Längsachse des Sohlenteils verlaufenden Fersenteil mit sich beidseitig der Längsachse erstreckenden Fußseitentcilen.

Die T-förmige Ausgestaltung des Fußteils ermöglicht durch ein Verschwenken der Fußseitenlaschen um die Längsachse des Sohlenenteils bzw. Wadentcils sowie ein Verschwenken des Fußsohlenteils um die Achse der Ferseneinschnürung eine einfache Überführung des Fußteils in eine Grundkonfiguration, in der nur noch relativ geringe Verformungen der Fußseitenlaschen zur Anpassung an die Fußkontur notwendig sind, um einen optimalen Sitz des Fußteils zu erzielen.

Wenn gemäß einer bevorzugten Ausführungsform die Fußseitenteile im Wesentlichen rechtwinklig zur Längsachse des Sohlenteils verlaufende Unterkanten und zu freien Seitenteilenden zu den Unterkanten hin geneigt verlaufende Oberkanten aufweisen, wird die Relativpositionierung der Fußseitenteile zum Fußsohlenteil und die Anpassung der Fußseitenteile an die Kontur des Fußes noch weiter vereinfacht.

Besonders positiv auf die einfache Verformbarkeit der Fußseitenteile zur Anpassung an die Fußflanken wirkt sich aus, wenn die Kissenkammer im Bereich der Fußseitenteile rahmenartig ausgebildet ist mit einer allseitig durch die Kissenkammer umgebenen Ausnehmung.

Eine erleichterte Anpassung des Formkissens an die Fersen- bzw. Sprunggelenkkontur wird möglich, wenn das Fersenteil in einem Fersenbereich mit einer Ausnehmung versehen ist.

Die Umformung der Wade beim Anlegen des Formkissens wird erleichtert, wenn die Kissenkammer im Bereich des Wadentcils einen U-förmigen Kissenbereich mit einer Kammerbasis und zwei Kammerschenkeln aufweist, wobei zwischen den Kammerschenkeln eine Schenkelverbindung vorgesehen ist.

Eine besonders effektive und zugleich einfache Ausgestaltung einer Formkissenhülle zur Erzielung eines auf das Formkissen wirkenden Formgebungseffektes wird möglich, wenn die Formkissenhülle zur Ausbildung und Trennung der Aufnahmebereiche eine untere Lage und eine obere Lage miteinander verbindende Verbindungsnähte aufweist.

Durch die Anordnung des Formkissens in der entsprechend zugeschnittenen Formkisscnhülle wird das Formkissen insgesamt in eine Grundkonfiguration überführt, auf der basierend nur noch relativ geringfügige Verformungen des Formkissens zur vollständigen Anpassung des Formkissens an die Körperteilkontur notwendig sind.

Im Fall der Ausbildung eines Ortheseninlays zur Unterschcnkelanordnung wird eine besonders weitgehende Anpassung des Fußteils des Formkissens an die Fußkontur wird möglich, wenn die Formkissenhülle neben einer Wadenteilaufnahme und einer Fußteilaufnahme zur Aufnahme des Wadenteils und des Fußteils des Formkissens eine Ausgestaltung der Fußteilaufnahme aufweist, bei der eine Sohlentasche zur Aufnahme des Sohlenteils mit zwei im Wesentlichen parallel und benachbart dazu verlaufenden Seitenteiltaschen zur Aufnahme der Fußseitenlaschen vorgesehen ist.

Eine bevorzugte Ausführungsform des Ortheseninlays wird nachfolgend anhand der Zeichnung näher erläutert. Es zeigen:
- **Fig. 1**: einen in der Ebene ausgebreiteten Kissenzuschnitt eines Formkissens;
- **Fig. 2**: eine Formkissenhülle zur Aufnahme des in **Fig. 1** dargestellten Formkissens;
- **Fig. 3**: ein durch Aufnahme des Formkissens in die Formkissenhülle gebildetes Ortheseninlay;
- **Fig. 4**: eine durch das Ortheseninlay ergänzte Schalenanordnung in einer Anlegekonfiguration;
- **Fig. 5**: die mit dem Ortheseninlay versehene Schalenanordnung in Tragekonfiguration;
- **Fig. 6**: eine Ventileinrichtung des Formkissens in Seitenansicht;
- **Fig. 7**: die in **Fig. 6** dargestellte Ventileinrichtung in Schnittdarstellung.

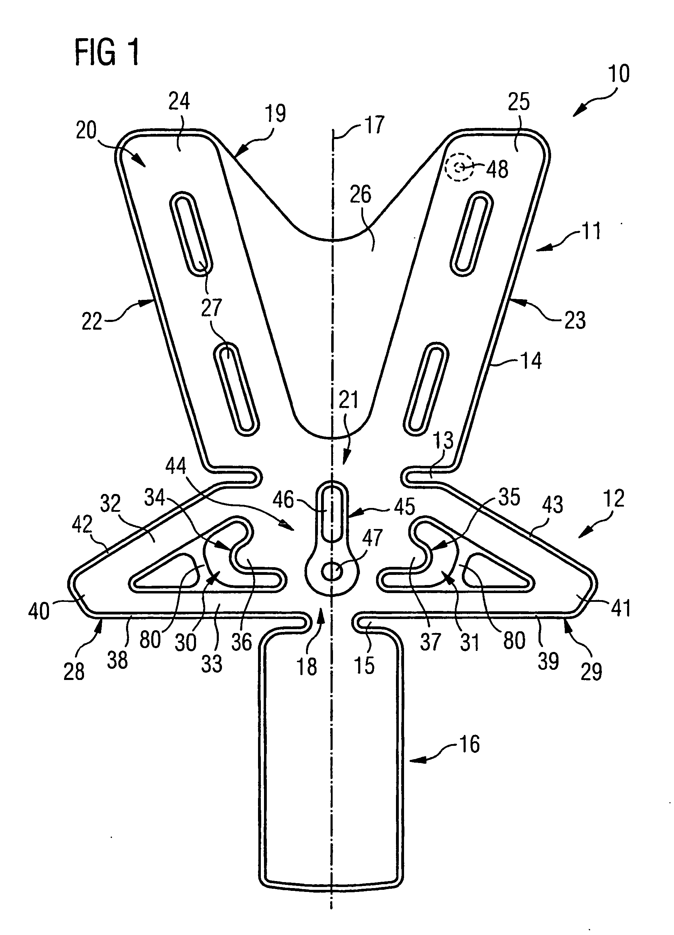

**Fig. 1** zeigt als ebenen Kissenzuschnitt ein Formkissen 10 mit einem Wadenteil 11 und einem Fußteil 12. Der Wadenteil 11 ist durch eine erste Einschnürung 13 in einer Kissenkontur 14 von dem T-förmig ausgebildeten Fußteil 12 abgeteilt. Der T-förmig ausgebildete Fußteil 12 ist seinerseits durch eine weitere, nachfolgend als Ferseneinschnürung 15 bezeichnete Einschnürung in einen Sohlenteil 16, der in Richtung und symmetrisch zu einer Längsachse 17 des Formkissens angeordnet ist, und einen quer zum Sohlenteil 16 verlaufenden und ebenfalls symmetrisch zur Längsachse 17 angeordneten Fersenteil 18 unterteilt.

Das in **Fig. 1** als ebener Kissenzuschnitt dargestellte Formkissen 10 besteht aus einer im vorliegenden Fall aus zwei Lagen aus thermoplastischem Kunststoff gebildeten gasdichten Hülle 19, die in Flächenbereichen sowie längs der Kissenkontur 14 zur Ausbildung einer gasdichten Kissenkammer 20 verschweißt ist. Im Bereich des Wadenteils 11 ist die Kissenkammer 20 U-förmig ausgebildet mit einer Kammerbasis 21 und im vorliegenden Fall divergierend von der Kammerbasis 21 abgehenden und im Wesentlichen V-förmig zueinander angestellten Kammcrschenkeln 22, 23. Zwischen den Kammerschcnkeln 22, 23 ist durch die - wie vorstehend erläutert - flächig miteinander verbundenen Kunststofflagen ein Verbindungsbereich 26 ausgebildet, der im Bereich von freien Schenkelenden 24, 25 der Kammerschenkel 22, 23 eine konkave Kontur aufweist. Um bei Anlage des Wadenteils 11 des Formkissens 10 am Unterschenkel eines Patienten eine möglichst gute Passform und Belüftung zu erreichen, sind die Kammerschenkel 22, 23 mit Ausnehmungen 27 versehen.

Der Fersenteil 18 des Fußteils 12 weist zwei sich jeweils von der Längsachse 17 des Formkissens 10 seitlich weg erstreckende Fußseitenteile 28, 29 auf, die jeweils eine Kissenausnchmung 30, 31 aufweisen, die durch einen rahmenartig ausgebildeten Bereich der Kissenkammer 20 umgeben sind. Zur relativen Stabilisierung von V-förmig zueinander angeordneten Kammerschenkeln 32, 33 der Fußseitenteile 28, 29 sind die Kammerschenkel 32, 33 über die jeweilige Kissenausnehmung 30, 31 hinweg mit Verbindungsstegen 80 verbunden. Darüber hinaus ist jeweils ein der Längachse 17 zugewandter, dem Fußknöchel zugeordneter Knöchelrandbereich 34, 35 der Kissenausnehmung 30, 31 zur Ausbildung einer in die Kissenausnehmung 30, 31 hineinragenden Kammerzunge 36, 37 konvex ausgebildet. Die Fußseitenteile 28, 29 weisen im vorliegenden Fall in etwa rechtwinklig zur Längsachse 17 verlaufende Unterkanten 38, 39 auf sowie zu freien Seitenteilenden 40, 41 hin geneigt auf die Unterkanten 38 bzw. 39 zulaufende Oberkanten 42, 43. In einem die beiden Fußseitenteile 28, 29 miteinander verbindenden Fersenbereich 44 ist eine weitere Kissenausnehmung 45 vorgesehen, die einen Langlochbereich 46 mit einem am unteren Ende des Langlochbereichs 46 ansetzenden und gegenüber dem Langlochbereich 46 verbreiterten Rundlochbereich 47 aufweist. Die Kissenausnehmung 45 ist damit entsprechend der Aufnahme des Fersenhöckers mit daran angrenzendem Achillessehnenbereich konfiguriert.

Abgeteilt durch die Ferseneinschnürung 15 erstreckt sich ausgehend vom Fersenbereich 44 des Fußteils 12 in Richtung der Längsachse 17 der in etwa rechteckförmig ausgebildete Sohlenteil 16 des Fußteils 12.

Wie aus **Fig. 1** ferner deutlich wird, ist im vorliegenden Fall die Kissenkammer 20 als eine über die verschiedenen Bereiche des Formkissens 10 hinweg kontinuierlich ausgebildete Kammer ausgestaltet, die über lediglich eine Ventileinrichtung 48 be- und entlüftet werden kann, die am Schenkelende 25 des Kammerschenkels 23 des Wadenteils 11 angeordnet ist. Zur Fixierung der nach Anpassung des Formkissens 10 am Unterschenkel eines Patienten eingestellten Form des Formkissens 10 ist in bekannter Weise die Kissenkammer 20 mit einer hier nicht näher dargestellten Formkörperfüllung aus einer Vielzahl von Formkörpern versehen, die nach Evakuierung der Kissenkammer 20 durch die Ventileinrichtung 48 ihre durch die Anpassung erzielte Relativanordnung beibehalten.

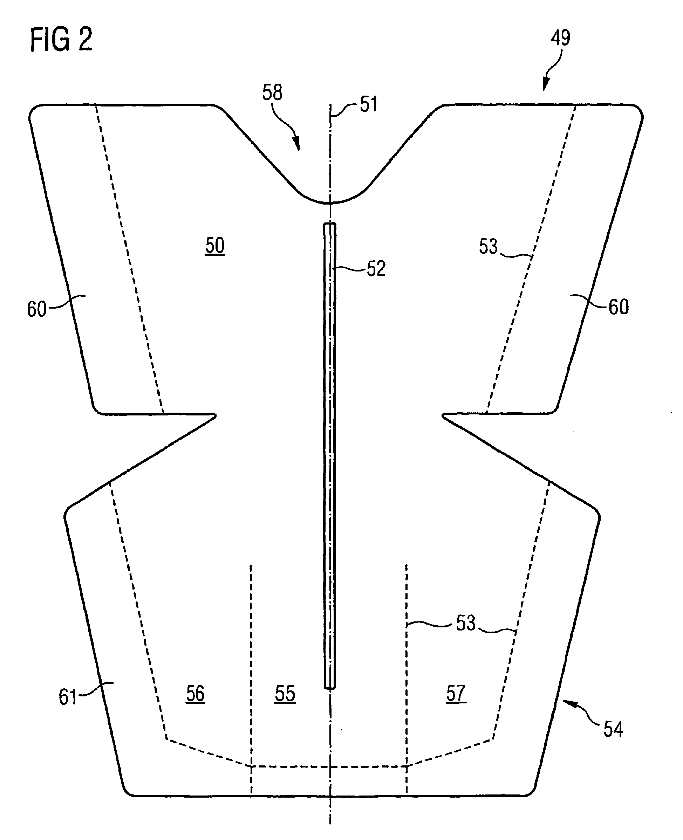

**Fig. 2** zeigt eine Formkissenhülle 49, die zur Aufnahme des in **Fig. 1** dargestellten Formkissens dient. Die Formkissenhülle 49 ist im vorliegenden Fall aus zwei miteinander vernähten Textillagen gebildet, wobei in der Darstellung gemäß **Fig. 2** lediglich die vordere Textillage 50 zu erkennen ist, die längs einer Längsachse 51 mit einem, beispielsweise durch einen nicht näher dargestellten Reißverschluss verschließbaren, Einsteckschlitz 52 versehen ist. Die rückwärtige und die in **Fig. 2** erkennbare vordere Textillage 50 weisen einen voneinander abweichenden Zuschnitt auf, der so gewählt ist, dass die Formkissenhülle 49 in einer Unterschenkelaufnahmekonfiguration, in der die Formkissenhülle 49 im Wesentlichen den Unterschenkel des Patienten umhüllend angeordnet ist, sowohl in der äußeren Textillage als auch in der inneren Textillage frei von Spannungen ist. Darüber hinaus weist die in **Fig. 2** dargestellte Formkissenhülle 49 durch zwischen den Textillagen ausgebildete Verbindungsnähte 53 abgenähte Bereiche auf, die in einer Fußteilaufnahme 54 der Formkissenhülle 49 zur Ausbildung einer Sohlentasche 55 und zweier jeweils seitlich der Sohlentasche 55 angeordneter und sich im Wesentlichen parallel hierzu erstreckender Seitenteiltaschen 56, 57 dienen.

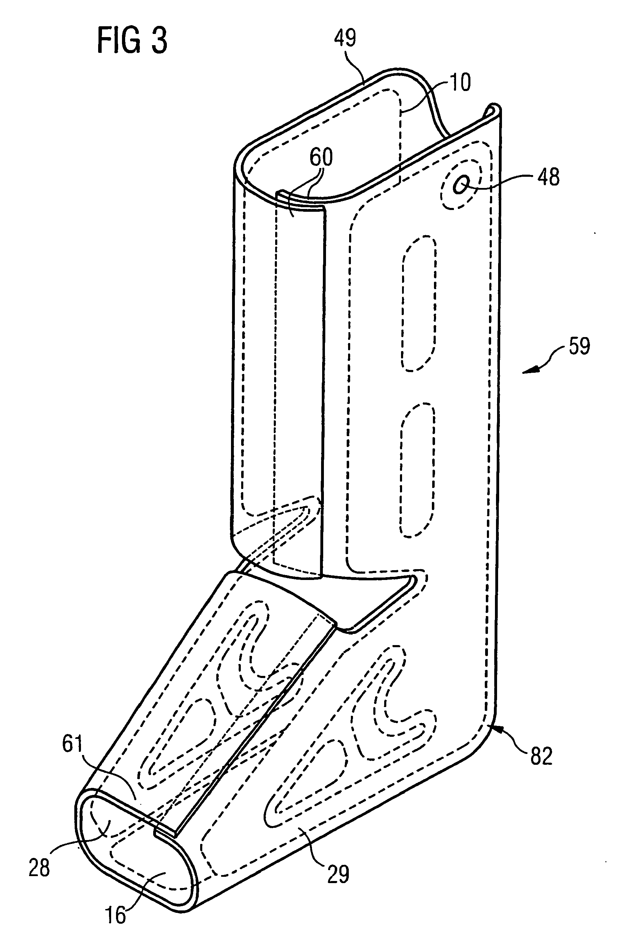

Wird nun das in **Fig. 1** dargestellte Formkissen durch den Einsteckschlitz 52 derart in die Formkissenhülle 49 eingebracht, dass der Wadenteil 11 des Formkissens 10 in einer oberhalb der Fußteilaufnahme 54 angeordneten Wadenteilaufnahme 58 angeordnet ist und der Fußteil 12 des Formkissens 10 in der Fußteilaufnahme 54 der Formkissenhülle 49 angeordnet ist, wobei das Sohlenteil 16 in die Sohlentasche 55 und die Fußseitenteile 28, 29 in die Seitenteiltaschen 56 und 57 eingeführt werden, so ergibt sich aufgrund des Gesamtzuschnitts der Formkissenhülle 49 und der im Wesentlichen parallel zueinander angeordneten Taschen, nämlich Seitenteiltaschen 56, 57 und Sohlentasche 55, in etwa die in Fig. 3 dargestellte Unterschenkelaufnahmckonfiguration des Formkissens 10 mit einem schalenförmigen Fußaufnahmebereich 82. Somit bildet das Formkissen 10 im Zusammenwirken mit der Formkissenhülle 49 ein Ortheseninlay 59. Dabei können die Wadenteilaufnahme 58 und die Fußteilaufnahme 54 begrenzende, abgenähte Randstreifen 60 und 61 einander zumindest teilweise überlappend angeordnet werden.

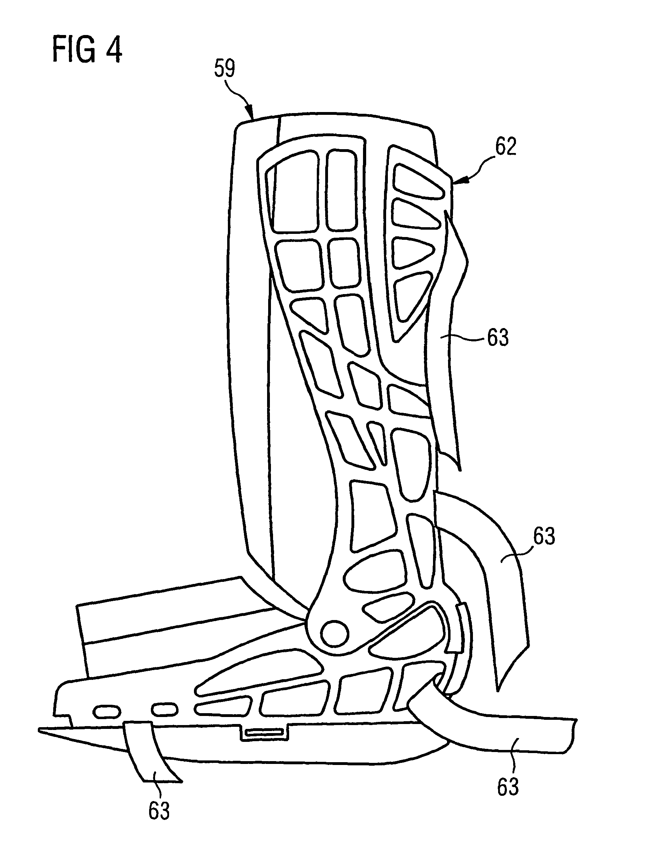

**Fig. 4** zeigt das in der Unterschenkelaufnahmekonfiguration angeordnete Ortheseninlay 59, das in eine Schalenanordnung 62, die zur fixierenden Aufnahme eines Unterschenkels dient, eingesetzt ist. Die durch das Ortheseninlay 59 ergänzte Schalenanordnung 62 kann nun am Unterschenkel des Patienten angeordnet und in dieser Relativposition durch Verschließen der hierfür vorgesehenen Verschlussbänder 63 gesichert werden.

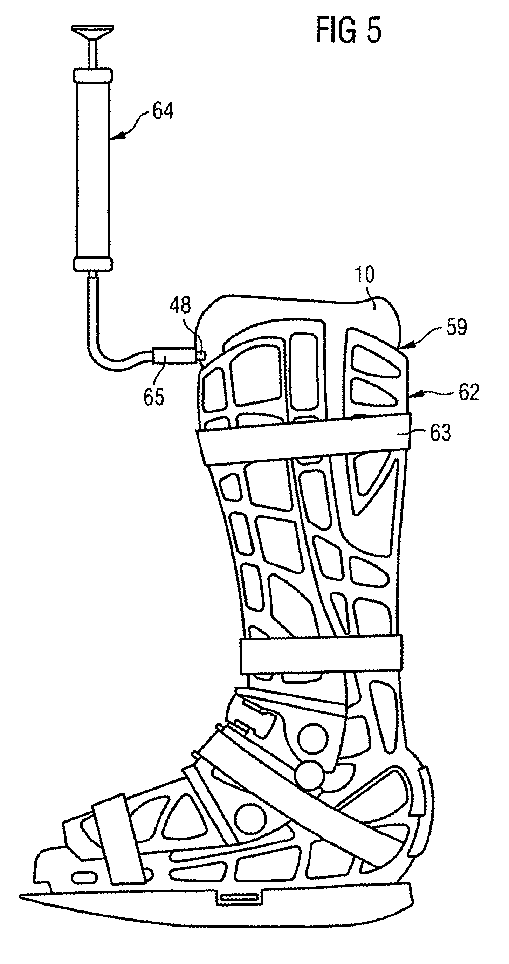

In der so realisierten, in **Fig. 5** dargestellten Tragekonfiguration der durch die Verschlussbänder 63 verschlossenen Schalenanordnung 62 erfolgt nun eine Evakuierung des Formkissens 10 über eine an die Ventileinrichtung 48 des Formkissens 10 angeschlossene Vakuumpumpe 64. Als Folge der Evakuierung des Formkissens 10 ergibt sich eine Fixierung der durch den Anlegevorgang des Formkissens 10 erreichten Relativanordnung der im Einzelnen nicht dargestellten Formkörper der Formkörperfüllung, so dass ein optimaler Sitz der Schalenanordnung 62 am Unterschenkel ermöglicht wird.

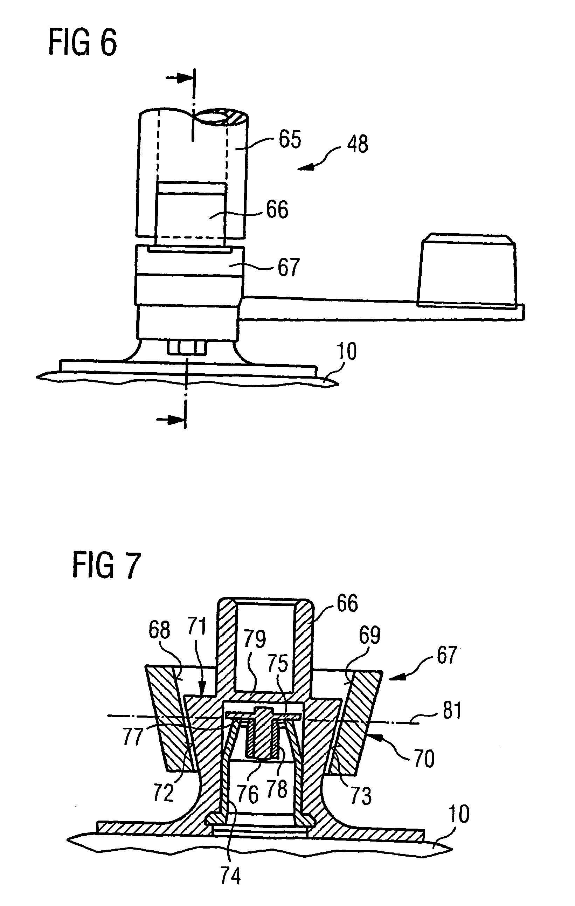

Dabei wird eine möglichst einfache Handhabung der in den **Fig. 6** und 7 dargestellten Ventileinrichtung 48 dadurch ermöglicht, dass bedingt durch das Aufschieben eines Schlauchmundstücks 65 der Vakuumpumpe 64 auf einen Ventilstutzen 66 der Ventileinrichtung 48 ein Klemmring 67 in eine Entlüftungsstellung verschoben wird, in der eine Evakuierung des Formkissens 10 über die Vakuumpumpe 64 erfolgen kann.

Wie in **Fig. 7** dargestellt, ist der Klemmring 67 auf seiner Innenwandung mit einem zwei Klemmringkeilflächen 68, 69 aufweisenden Keilprofil 70 versehen, das mit einem Keilprofil 71 am Ventilstutzen 66 zusammenwirkt, welches zwei den Klemmringkeilflächen 68, 69 komplementär zugeordnete Stutzenkeilflächen 72, 73 aufweist.

Im Ventilstutzen 66 befindet sich ein Ventileinsatz 74, der zur Aufnahme einer Membranscheibe 75 dient, die mit einem Führungszapfen 76 axial verschiebbar in einer an einem Diehtrand 77 des Ventileinsatzes 74 ausgebildeten Membranführung 78 geführt ist. In der in **Fig. 7** dargestellten Dichtstellung liegt die Membranscheibe 75 durch einen im Inneren des Formkissens 10 ausgebildeten Unterdruck abdichtend am Dichtrand 77 an und dient somit während der Evakuierung auch als Rückschlagventil. In dieser Stellung ist es möglich, durch einen durch die Vakuumpumpe 64 auf die Membranscheibe 75 wirkenden Unterdruck die Membranscheibe 75 bis zur Anlage gegen einen im Ventilstutzen 66 ausgebildeten Anschlagsteg 79 aus der Membranführung 78 herauszubewegen, um den Unterdruck im Formkissen 10 zu erzeugen oder zu vergrößern.

Wenn aus der in **Fig. 7** dargestellten Dichtstellung der Klemmring 67 axial nach oben in die Belüftungsstellung bewegt wird, üben die Klemmringkeilflächen 68, 69 des Klemmrings 67 eine Klemmkraft auf die Stutzenkeilflächen 72, 73 des Ventilstutzens 66 aus, mit der Folge, dass sich der aus einem formelastischen Kunststoffmaterial gebildete Ventilstutzen 66 im Bereich einer Membranebene 81, in der sich die Membranscheibe 75 befindet, verformt, so dass durch eine hiermit verbundene Querschnittsverringerung des Ventilstutzens 66 eine Verformung der Membranscheibe 75 aus der Membranebene 80 erfolgt und keine abdichtende Anlage der Mcmbranscheibc 75 gegen den Dichtrand 77 des Vcntileinsatzes 74 mehr gegeben ist. Hierdurch kann von außen Umgebungsluft in das Formkissen 10 zur Belüftung des Formkissens 10 einströmen.

## Patentansprüche

1. Ortheseninlay zur Anordnung in einer Stützschalenanordnung einer Körperteilorthese mit einem Formkissen zur Anformung an den Körperteil mit zumindest einer in einer gasdichten Hülle ausgebildeten Kissenkammer, die über eine Ventileinrichtung evakuierbar ist und eine Formkörperfüllung aus einer Vielzahl von Formkörpern aufweist, und mit einer Formkissenhülle zur Aufnahme des Formkissens,
wobei das Formkissen (10) einen ebenen Kissenzuschnitt mit einem Basisteil (18) mit zumindest zwei Seitenteilen (28, 29) und einem vom Basisteil über eine Einschnürung (15) abgeteilten Laschenteil (16) umfasst, und die Formkissenhülle (49) zumindest drei jeweils den Seitenteilen des Basisteils und dem Laschenteil zugeordnete Aufnahmebereiche, nämlich zwei Seitenteilaufnahmen (56, 57) und eine Laschenteilaufnahme (55), aufweist, die benachbart zueinander angeordnet sind, **dadurch gekennzeichnet, dass** die Seitenteile und das Laschenteil des Formkissens nach Aufnahme in den zugeordneten Aufnahmebereichen der Formkissenhülle eine schalenförmige Körperteilaufnahme zur zumindest anteiligen Umhüllung des aufzunehmenden Körperteils bilden.

2. Ortheseninlay zur Anordnung an einem Unterschenkel nach Anspruch 1,
**dadurch gekennzeichnet,**
**dass** der Kissenzuschnitt in einen Wadenteil (11) und einen Fußteil (12) unterteilt ist, wobei der Fußteil T-förmig ausgebildet ist mit einem der Fußsohle zugeordneten Sohlenteil (16) und einem durch eine Ferseneinschnürung (15) vom Sohlenteil abgeteilten und quer zu einer Längsachse (17) des Sohlenteils verlaufenden Fersenteil (18) mit sich beidseitig der Längsachse erstreckenden Fußseitenteilen (28, 29).

3. Ortheseninlay nach Anspruch 2,
**dadurch gekennzeichnet,**
**dass** die Fußseitenteile (28, 29) im Wesentlichen rechtwinklig zur Längsachse (17) des Sohlenteils (16) verlaufende Unterkanten (38, 39) und zu freien Seitenteilenden (40, 41) zu den Unterkanten hin geneigt verlaufende Oberkanten (42, 43) aufweisen.

4. Ortheseninlay nach Anspruch 2 oder 3,
**dadurch gekennzeichnet,**
**dass** die Kissenkammer (20) im Bereich der Fußseitenteile (28, 29) rahmenartig ausgebildet ist mit einer allseitig durch die Kisscnkammer umgebenen Ausnehmung (30, 31).

5. Ortheseninlay nach einem der Ansprüche 2 bis 4,
**dadurch gekennzeichnet,**
**dass** der Fersenteil (18) in einem Fersenbereich (44) mit einer Ausnehmung (45) versehen ist.

6. Ortheseninlay nach einem der Ansprüche 2 bis 5,
**dadurch gekennzeichnet,**
**dass** die Kissenkammer (20) im Bereich des Wadenteils (11) einen U-förmigen Kissenbereich mit einer Kammerbasis (21) und zwei Kammerschenkeln (22, 23) aufweist, wobei zwischen den Kammerschenkeln eine Schenkelverbindung (26) vorgesehen ist.

7. Ortheseninlay nach Anspruch 1,
**dadurch gekennzeichnet,**
**dass** die Formkissenhülle (49) zur Ausbildung und Trennung der Aufnahmebereiche (55, 56, 57) eine untere Lage und eine obere Lage miteinander verbindende Verbindungsnähte (53) aufweist.

8. Ortheseninlay zur Anordnung an einem Unterschenkel nach Anspruch 1,
**dadurch gekennzeichnet,**
**dass** die Formkissenhülle (49) eine Wadenteilaufnahme (58) und eine Fußteilaufnahme (54) zur Aufnahme eines Wadenteils (11) und eines Fußteils (12) des Formkissens (10) aufweist, wobei die Fußteilaufnahme eine Sohlentasche (55) zur Aufnahme eines Sohlenteils (16) mit zwei im Wesentlichen parallel und benachbart dazu verlaufenden Seitenteiltaschen (56, 57) zur Aufnahme von Fußseitenteilen (28, 29) aufweist.

## Claims

1. An orthotic inlay for arrangement in a supporting shell configuration of an orthotic device for a body part having a moulded cushion for shaping to the body part, with at least one cushion chamber formed in an airtight casing, said chamber being evacuable via a valve mechanism and having a moulded body filling consisting of a plurality of moulded bodies and having a moulded cushion casing to accommodate the moulded cushion, the moulded cushion (10) having a planar cushion blank with a base part (18) with at least two side parts (28, 29) and a strap part (16) which is separated from the base part by a constriction (15), and the moulded cushion casing (49) having at least three receptacle areas assigned to the side parts of the base part and the strap part, namely two side part receptacles (56, 57) and one strap part receptacle (55), which are arranged adjacent to one another
**characterized in that**
after being accommodated in the respective receptacle areas of the moulded cushion casing the side parts and the strap part of the moulded cushion form a shell-like body part receptacle for enclosing at least a portion of the body part to be accommodated.

2. Orthotic inlay for arrangement on a lower leg according to Claim 1,
**characterized in that**
the cushion blank is subdivided into a calf part (11) and a foot part (12), whereby the foot part is designed to be T-shaped, with a sole part (16) assigned to the sole of the foot and a heel part (18) which runs across the longitudinal axis (17) of the sole part and is divided from the sole part by a heel constriction (15), with foot side parts (28, 29) extending on both sides of the longitudinal axis.

3. Orthotic inlay according to Claim 2,
**characterized in that**
the foot side parts (28, 29) have lower edges (38, 39) running essentially perpendicular to the longitudinal axis (17) of the sole part (16) and upper edges (42, 43) running to the free side part ends (40, 41) with an inclination toward the lower edges.

4. Orthotic inlay according to Claim 2 or 3,
**characterized in that**
the cushion chamber (20) is designed like a frame in the area of the foot side parts (28, 29) with a recess (30, 31) surrounded by the cushion chamber on all sides.

5. Orthotic inlay according to one of Claims 2 through 4,
**characterized in that**
the heel part (18) is provided with a recess (45) in a heel area (44).

6. Orthotic inlay according to one of Claims 2 through 5,
**characterized in that**
the cushion chamber (20) has a U-shaped cushion area in the area of the calf part (11) with a chamber base (21) and two chamber legs (22, 23), with a leg connection (26) being provided between the chamber legs.

7. Orthotic inlay according to Claim 1,
**characterized in that**
the moulded cushion casing (49) has connecting seams (53) which join a lower layer and an upper layer to form and separate the receptacle areas (55, 56, 57).

8. Orthotic inlay for arrangement on a lower leg according to Claim 1,
**characterized in that**
the moulded cushion casing (49) has a calf part receptacle (58) and a foot part receptacle (54) to accommodate a calf part (11) and a foot part (12) of the moulded cushion (10), whereby the foot part receptacle has a sole pocket (55) to accommodate a sole part (16) with two side part pockets (56, 57) running essentially parallel and adjacent to the former to accommodate the foot sides parts (28, 29).

## Revendications

1. Insert d'orthèse destiné à être disposé dans un dispositif de coque de soutien d'une orthèse pour une partie du corps, avec un coussin moulé destiné à épouser la partie du corps, avec au moins un compartiment pour le coussin réalisé dans une enveloppe étanche au gaz, qui est évacuable par l'intermédiaire d'un dispositif de soupapes et qui comporte un remplissage par corps moulés composé d'une pluralité de corps moulés et avec une enveloppe de coussin moulé pour le logement du coussin moulé,
le coussin moulé (10) comprenant une pièce découpée de coussin plane comportant une partie de base (18) avec au moins deux parties latérales (28, 29) et une patte (16) délimitée de la partie de base par un collet (15), et l'enveloppe de coussin moulé (49) comportant trois zones de logement dont chacune est associée aux parties latérales de la partie de base et à la patte, à savoir trois logements pour parties latérales (56, 57) et un logement de patte (55), qui sont voisins l'un de l'autre,
**caractérisé en ce qu'**
après avoir été logées dans les zones de logement associées de l'enveloppe de coussin moulé, les parties latérales et la patte du coussin moulé forment un logement en forme de coque pour la partie du corps, pour envelopper au moins partiellement la partie du corps à recevoir.

2. Insert d'orthèse destiné à être placé sur une jambe selon la revendication 1,
**caractérisé en ce que**
la pièce découpée de coussin est divisée en une partie mollet (11) et en une partie pied (12), la partie pied étant réalisée en forme de T, avec une partie semelle (16) associée à la plante du pied et avec une partie talon (18) délimitée de la partie semelle par un collet de talon (15) et s'étendant à la transversale d'un axe longitudinal (17) de la partie semelle, avec des parties latérales du pied (28, 29) s'étendant de part et d'autre de l'axe longitudinal.

3. Insert d'orthèse selon la revendication 2,
**caractérisé en ce que**
les parties latérales du pied (28, 29) comportent des arêtes inférieures (38, 39) s'étendant sensiblement à angle droit par rapport à l'axe longitudinal (17) de la partie semelle (16) et des arêtes supérieures (42, 43) inclinées vers les arêtes inférieures, vers des extrémités libres des parties latérales (40, 41).

4. Insert d'orthèse selon la revendication 2 ou 3,
**caractérisé en ce que**
dans la zone des parties latérales du pied (28, 29), le compartiment de coussin (20) est réalisé sous la forme d'un cadre avec un évidement (30, 31) entouré de toutes parts par le compartiment de coussin.

5. Insert d'orthèse selon l'une quelconque des revendications 2 à 4,
**caractérisé en ce que**
dans une zone de talon (44), la partie talon (18) est munie d'un évidement (45).

6. Insert d'orthèse selon l'une quelconque des revendications 2 à 5,
**caractérisé en ce que**
le compartiment de coussin (20) comporte dans la zone de la partie mollet (11) une zone de coussin en forme de U avec une base de compartiment (21) et avec deux branches de compartiment (22, 23), un assemblage des branches (26) étant prévu entre les branches du compartiment.

7. Insert d'orthèse selon la revendication 1,
**caractérisé en ce que**
pour former et pour séparer les zones de logement (55, 56, 57), l'enveloppe de coussin moulé (49) comporte des soudures d'assemblage (53) reliant une couche inférieure et une couche supérieure.

8. Insert d'orthèse destiné à être disposé sur une jambe selon la revendication 1,
**caractérisé en ce que**
l'enveloppe de coussin moulé (49) comporte un logement pour la partie mollet (58) et un logement pour la partie pied (54), destinés au logement d'une partie mollet (11) et d'une partie pied (12) du coussin moulé (10), le logement pour la partie pied comportant une poche pour semelle (55) destinée au logement d'une partie semelle (16) avec deux poches pour parties laterales (56, 57) destinées au logement de parties latérales du pied (28, 29) et s'étendant essentiellement en parallèle et au voisinage de la poche pour semelle.
